# EUROPEAN PATENT SPECIFICATION

(11) **EP 4 177 903 B1**
(45) Date of publication and mention of the grant of the patent: **10.06.2026**
(21) Application number: 22185079.5
(22) Date of filing: 15.07.2022
(51) Int. Cl.: G16H 20/30, G16H 50/20, G16H 50/30, G16H 50/70, A61B 5/00, H04W 84/18

(54) **BODY AREA NETWORK HAVING SENSING CAPABILITY**
KÖRPERBEREICHSNETZWERK MIT ERFASSUNGSFÄHIGKEIT
RÉSEAU CORPOREL DOTÉ D'UNE CAPACITÉ DE DÉTECTION

(30) Priority: 13.10.2021 US 202163255166 P
(43) Date of publication of application: 10.05.2023
(73) Proprietor: Zimmer, Inc., Warsaw, IN 46580 (US)
(72) Inventor: BRINCAT, Mark, Moreton in Marsh, GL56 0BD (GB); AMIOT, Louis-Philippe, Montreal, H4A2L5 (CA); GALLOWAY, Annelise, Winona Lake, 46590 (US); LORENTZ, Jayme, Warsaw, 46580 (US); SPOONER, Ted, Grand Rapids, 49506 (US)
(74) Representative: Taor, Simon Edward William

(56) References cited:
- US-A1- 2013 095 459
- US-A1- 2016 302 721
- US-A1- 2021 282 652
- US-B1- 10 076 286

## Description

### BACKGROUND

Patients may ultimately require surgical intervention. For example, when pain becomes unbearable for an individual, surgery may be recommended. However, such surgical intervention occurs when it is typically already too late to facilitate other non-surgical outcomes. Prior to surgical intervention, users may try preoperative measures such as physical or occupational therapy. However, current techniques involving physical therapy or occupational therapy do not monitor or adequately assess user range of motion, identify user movement and/or anatomical trends, do not assess the user for signs of fatigue before surgical intervention, and do not monitor patient progress after surgical intervention.

US2013095459A1 discloses a monitoring system for a person, including a processor coupled to one or more wireless nodes; a wearable mobile appliance in communication with the client and one or more wireless nodes; and one or more computer implemented agents with rules executed by the processor, the rules being selected to respond to a client communication relating to a predetermined health condition, each agent communicating with another computer implemented agent, the client or the treatment professional, and upon receiving a communication from the client, the processor selecting one or more computer implemented agents to reply with an instruction on healthy client behavior.

### SUMMARY

According to an aspect, there is provided a system for monitoring health of a user as set out in claim 1. Optional features are set out in claims 2 to 8.

According to another aspect, there is provided a method for monitoring health of a user as set out in claim 9. Optional features are set out in claims 10 to 11.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 illustrates a system including a network of sensor devices implanted within or worn by a user in accordance with at least one example of the present application.
FIG. 2 shows exemplary sensors worn by the user in accordance with at least one example of the present application.
FIG. 3 shows further exemplary sensors worn by the user in accordance with at least one example of the present application.
FIGS. 4A and 4B shows a first sensor implanted adjacent the knee joint of a user having a passive component and an active sensing component in accordance with at least one example of the present application.
FIG. 5 illustrates a sensor data processing system in accordance with at least one example of the present application.
FIG. 6 illustrates a communication diagram for the sensor data processing system in accordance with at least one example of the present application.
FIG. 7 illustrates a machine learning engine for determining feedback in accordance with at least one example of the present application.
FIG. 8 illustrates a flowchart showing a method for monitoring health of a patient and providing the patient with health advice using predictive analytics; the method including determining what device to use to collect data for the predictive analytics in accordance with at least one example of the present application.
FIG. 9 illustrates a flowchart showing a second method for monitoring health of a patient and providing the patient with health advice using predictive analytics; the method including determining what device to use to collect data for the predictive analytics in accordance with at least one example of the present application
FIG. 10 illustrates a block diagram of an example machine upon which any one or more of the techniques, systems or methods discussed herein may perform in accordance with at least one example of this disclosure.

### DETAILED DESCRIPTION

Apparatuses, systems and techniques described herein may be used for providing preoperative and proactive feedback to a user and/or health care provider. The preoperative feedback may include a personalized intervention recommendation for the user that can be used to preemptively address one or more health conditions prior to or in lieu of a surgical intervention for the user. This personalized intervention recommendation can be related to overall user health, user joint health, or other user criteria or conditions such as but not limited to morbidity conditions, a fatigue of the user, a pain level identified by the user, a range of motion of the user, a user exercise score, a user's progress towards a goal defined by the user, a body orientation of the user based upon at least the first sensor, or a measured physiological characteristic of the user. For example, the personalized intervention recommendation can include information related to fatigue level, pain management, a physical therapy plan, a range of motion plan (stretching plan), an exercise plan or goal, a recommendation to limit or increase movement speed or acceleration, a recommendation to limit certain movement(s) due to fatigue or injury, or the like. The personalized intervention recommendation can be altered or updated with input from the user such as by updating a fitness goal, life plan or the like.

Systems and techniques described herein may be used to monitor user progress, provide the user with health updates, and modify user behavior including by modifying a health goal or health plan. A predictive analytics model may be utilized to provide the personal intervention recommendation. This predictive analytics model can be trained (e.g., using machine learning techniques as described herein) to predict health outcomes related to the user using the user's own personalized data. For example, an electronic device such as an electronic controller, control circuitry or the like, may receive data generated by a sensor implanted in the user. Further data can optionally be received by the electronic device from a network of sensors (e.g., implanted ("implants") or worn by the user) if desired. Using the data (combined data, data from the single implant or data from one or more implants or wearables) the electronic device may generate the predictive analytics model using machine learning or other technique. The electronic device may use the predictive analytics model (which may be personalized to the user) to output the personalized intervention recommendation to the user. In some examples, separate (but optionally related) predictive analytics models may be stored in memory and used by the electronic device.

In an example, the predictive analytics model may differ based on the type user data collected. As an example, the predictive analytics model may differ if data is gathered from an implant in the knee of the user as opposed to if the data was gathered from a sensor worn on a shoulder or back of the user. Different machine learning models may be trained using different sensor data. In addition to type of sensor data, quality and/or sufficiency of sensor data may be used to select one of the machine learning models according to some examples. In yet further examples, sensor data can be combined to provide for sufficiency to perform the predictive analytics model.

In some examples the sensors discussed herein may include memory, communication circuitry, or processing circuitry (which may include an integrated circuit, such as a system on a chip, a field-programmable gate array (FPGA), a processor, etc.). The sensors may be used to generate, store, or send data. However, such storing may not be applicable in all cases. Furthermore, as discussed some sensors contemplated include a passive component as will be discussed in further detail.

In an example, the predictive analytics model generated or using the sensor data may be used to detect and predict outcomes related to user-specific health or life goals (e.g., "I want to play 18 holes of golf," "I want to be able to bike 10 miles," "I want to play with my grandkids," "I want to be able to walk 8 miles a day on my trip in Europe this summer" etc.). The user-specific goal can be related to personalized intervention recommendations for the user that can be used to preemptively address one or more health conditions. The health goal can be converted into a set of one or more metrics. The sensor data and the analytics predictive model may be used to determine whether metrics of the set of one or more metrics are satisfied. In response to determining that all metrics of the set of one or more metrics are satisfied, an indication that the user-specific goal has been achieved may be output (e.g., a message on a user interface, audio, or the like, such as indicating "congratulations, you have met your goal and now may do activity X"). In response to determining that a metric of the set of one or more metrics is not satisfied, or a health risk is identified as possibly being close to being triggered, an indication corresponding to the metric or warning may be output (e.g., encouragement or details about how to achieve the goal, additional education information such as "improve walking gate by lengthening stride", "Warning: you are overly fatigued, do not perform any strenuous activity at this time" or the like).

These systems and techniques may improve user outcomes which can preemptively address one or more health conditions prior to or in lieu of a surgical intervention for the user. Using the sensors as a network, intelligent data may be collected with a tiered architecture for processing the data. The systems and techniques described herein may be used to validate and improve accuracy of data sources, activate real-time follow-up, configure event-based changes to data collection frequency and/or data collection type and fidelity (e.g., in response to a detected drop in walking speed), or the like. In some examples, an application (e.g., an app) may be used, such as on a mobile device to push a request to one or more of the sensors to increase or decrease a frequency of data collection or transmission. In an example, the sensor may be dormant, and activated (e.g., using information generated by a gyroscope or by activating a gyroscope) based upon need including that quality or sufficiency of data from another sensor implanted or worn by the user is insufficient.

In some examples, battery life may be considered or sacrificed based on current needs. A finite battery life of an implanted sensor may be managed. For example, the implanted sensor may be placed into a sleep or semi-sleep mode, where the sensor only checks periodically for data or only periodically outputs data (e.g., every hour, day, 15 minutes, etc.). The length of time between periods may be adjusted in accordance with user need (e.g., data collected from a wearable device regarding user posture was insufficient) and/or battery life management.

In an example, data may be collected locally at a mobile device (e.g., a smart phone), such as to build a user profile (e.g., a user-specific model). The user profile may be based upon the predictive analytic model and the personalized intervention recommendation. When one or more of the sensors communicates with the mobile device, the mobile device may send a message to the implanted sensor to change modes (e.g., wake up into a more intense data collection mode, download a software or firmware update, etc.).

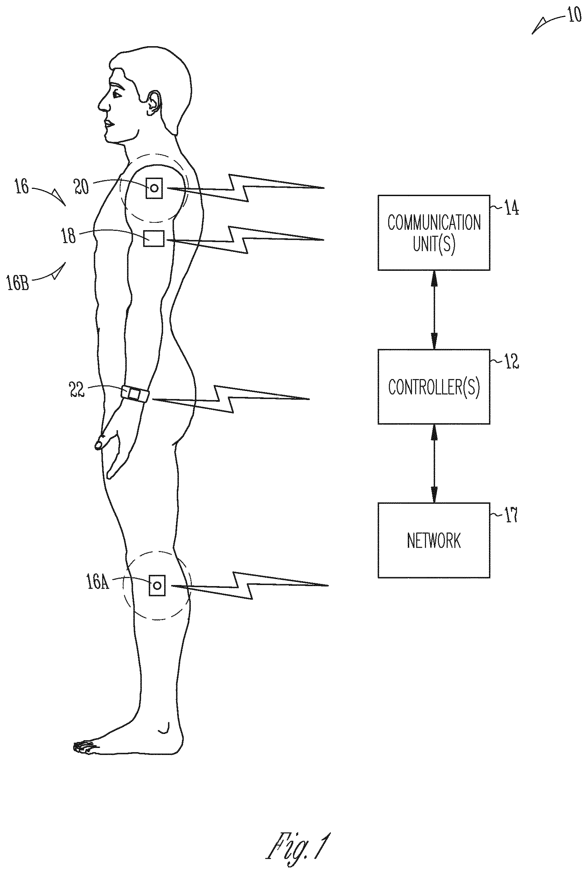

FIG. 1 shows a system 10 for sensor data processing that includes a controller 12 (e.g., processor, processing circuitry, memory, software, etc.), a communication unit 14 and a plurality of sensors 16. Optionally, the system 10 can communicate with a network 17 of other devices. The plurality of sensors 16 can include at least one implanted sensor 16A and one or more sensors on wearable devices 16B. The wearable devices 16B can include, but are not limited to, a patch 18, a shoulder mounted sensor 20 and a smart watch 22.

The system 10 can include an example architecture and componentry for a computer-implemented system. The controller 12 can include memory implement various algorithms. According to some examples, the controller 12 can be configured as a client that can run portions or all of the sensor data processing discussed herein. The controller 12 can be patient, clinician, or healthcare provider electronic devices for monitoring and/or collecting data locally or remotely from the plurality of sensors 16 and/or collecting data from or otherwise communicating with a server and/or data repository via the network 17. The controller 12 can be part of the server and/or data repository. The controller 12 can store and execute the sensor data assessment system. The controller 12 can be associated with and used for multiple data storage functions. Algorithms implemented by the controller 12 may be performed on circuitry (e.g., a processor, software, firmware, hardwired circuitry, etc.) that is capable of performing various functions. The controller 12, the plurality of sensors 16 and/or other system components not specifically shown (e.g., data repository, server, etc.) can be configured to communicate with one another such as via the communication unit 14 and/or can execute functions alone or in conjunction with one another over network 17. The controller 12 can include any number of different portable electronic mobile devices, including, e.g., cellular phones, personal digital assistants (PDA's), laptop computers, portable gaming devices, portable media players, e-book readers, watches, as well as non-portable devices such as desktop computers. The controller 12 can include one or more input/output devices configured to allow user interaction with one or more programs. In one example, the controller 12 can run a web browser that accesses/executes and presents a web application for use by the user. In another example, the controller 12 can execute an application outside of a web browser, e.g. an operating system specific application that accesses/executes and presents a native OS application for use by the user.

Network 17 can include one or more terrestrial and/or satellite networks interconnected to provide a means of communicatively connecting the controller 12 and other system components. In one example, network 17 can be a private or public local area network (LAN) or Wide Area Network (WANs). Network 17 can include both wired and wireless communications according to one or more standards and/or via one or more transport mediums. In one example, network 17 includes wireless communications according to one of the 802.11 or Bluetooth specification sets, or another standard or proprietary wireless communication protocol. Network 17 can also include communications over a terrestrial cellular network, including, e.g. a GSM (Global System for Mobile Communications), CDMA (Code Division Multiple Access), EDGE (Enhanced Data for Global Evolution) network. Data transmitted over network 17, e.g., from the plurality of sensors 16 to the controller 12 via the communication unit 14 can be formatted in accordance with a variety of different communications protocols. For example, all or a portion of network 17 can be a packet-based, Internet Protocol (IP) network that communicates data in Transmission Control Protocol/Internet Protocol (TCP/IP) packets, over, e.g., Category 5, Ethernet cables. The network 17 can be configured in a mesh configuration or a spoke and hub configuration with the controller.

The memory of the controller 12 and/or sensors 16 (or other system components) can include, e.g., a standard or proprietary electronic database or other data storage and retrieval mechanism. In one example, memory includes one or more databases, such as relational databases, multi-dimensional databases, hierarchical databases, object-oriented databases, or one or more other types of databases. Memory can be implemented in software, hardware, and combinations of both. In one example, memory include proprietary database software stored on one of a variety of storage mediums on a data storage server connected to network 17 and configured to store data such as measured/collected pre-operative sensor data or other information. Storage media included in or employed in cooperation with memory can include, e.g., any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media.

The controller 12 can employ memory to store and retrieve pre-operative sensor data. Additionally, the controller 12 can store and retrieve data or other information from analytics executed on sensor data and/or a patient plan or wellness goal, as well as data and other information related to patient population modeling.

The plurality of sensors 16 can gather data regarding the user. The plurality of sensors 16 are networked and can communicate electronically (e.g., wirelessly) with the communication unit 14. Communication unit 14 can be, for example, a receiver, a transceiver, or other communication circuitry (e.g., Bluetooth Low Energy), 3GPP LTE, WiFi, near field communication (NFC), another healthcare compliant communication protocol, or the like. The communication unit 14 can include parts that are part of the plurality of sensors 16 according to some examples. Communication unit 14 can also electronically communicate with the controller 12. Communication unit 14 can be one or more devices worn or in proximity to the patient at different times, for example a smartphone, smart phone, tablet, connected (wireless) exercisie equipment, smartwatch, etc. For example, the smartwatch can double as a sensor and communication unit 14.

The plurality of sensors 16 can electronically communicate with one another according to some examples. For example, the smart watch 22 can electronically communicate with one or more of the one implanted sensor 16A, the patch 18, or the shoulder mounted sensor 20. The shoulder mounted sensor 20 can give information on user posture, this can be transmitted to the controller 12 and/or others of the plurality of sensors 16 such as the implanted sensor 16A. Posture data could inform data coming from the knee regarding range of motion, for example.

At least one of the shoulder mounted sensor 20, the implanted sensor 16A, the patch 18 and/or the smart watch 22 can include an inertial measurement unit (IMU) gathering vector and acceleration information such as by a gyroscope and an accelerometer as known in the art. Examples of various sensors are described in prior applications such as U.S. Patent Application Publication Nos. 2020/0335222A1, 2021/0065870A1, 20190272917A1 and U.S. Patent No. 10,561,360. Using the IMU data, user posture and other relevant data relating to movement of the patient can be gathered.

The implanted sensor 16A (e.g., a first sensor) can be associated with a knee joint of the patient. However, other examples contemplate the implanted sensor 16A can be implanted in other locations in the body. Wearable devices 16B worn in any location not just the locations illustrated. The plurality of sensors 16 depicted in FIGS. 1-3 are merely illustrative and other sensors in other locations can be used in examples according to this disclosure. The patch 18 can provide a therapy (e.g., hormones, insulin, or the like) to the patient in addition to mounting a sensor. Thus, the patch 18 could be multi-function, i.e. also be a nicotine patch/hormonal therapy/glucose monitoring in addition to sensing such as with IMU. In one example, the smart watch 22 can be an off-the-shelf consumer wearable device such as, for example, Fitbit, Jawbone, Apple Watch, or other consumer wearable electronic devices. However, it is contemplated the smart watch 22 can be a custom sensor that is configured to be worn by the user to collect pre-operative health data. It is contemplated wearable devices 16B with sensors can be attached via a garment (e.g., smart clothes), harness, patch, strap, temporary adhesive or other mechanism known in the art.

The implanted sensor 16A can be a knee sensor having a small form factor and can be installed during a minimally invasive procedure on the knee joint such as during a knee arthroscopy surgery (scope) to repair torn cartilage or access knee cartilage damage, for example. The implanted sensor 16A can be no bigger than an Radiostereometric analysis (RSA) bead, for example. The implanted sensor 16A (or sensors) can be configured to monitor steps, forces, friction, temperature, or the like. The implanted sensor 16A can provide useful data from positions throughout the body not just the knee joint. For example, the implanted sensor 16A can be embedded in the shoulder, feet or hip. In some examples, the implanted sensor 16A may be attached directly to the bone or be inserted through a bone.

The plurality of sensors 16 can comprise a network or array according to the present disclosure. The plurality of sensors 16 can include any one or combination of an IMU, an accelerometer, a temperature sensor, a force sensor, a resistance sensor, a tachometer, a healing indicator, a pH measure sensor, a tension or compression sensor, callous formation sensing tape, a strain sensor (e.g., strain gauge), a gyroscope, a moisture (sweat) sensor, or the like. The sensors can implanted or part of a wearable device as discussed and shown. The plurality of sensors can be data collection devices and may be part of a watch, fitness tracker, a wrist-worn device, a sweat monitor (e.g., electrolytes level), a blood-sugar monitor (e.g., for diabetes), a heart monitor (e.g., EKG or ECG), a heart rate monitor, a pulse oximeter, a stress level monitor (e.g., via an Apple watch), a respiratory rate monitor device, a 'life-alert,' an ear wearable (e.g., for measuring intercranial pressure, such as via the tympanic membrane), a head attached wearable, an ultrasound wearable, a microphone dysphonia device, an augmented reality device (e.g., the HoloLens or other AR/MR or VR devices, such as for measuring dizziness, gait, etc.), an implantable sensor, a traditional medical device implant, on-body, or attached devices (e.g., a dialysis machine, heart devices such as pacemaker, defibrillator, etc., CPAP, blood-sugar testing devices, drug tests, etc.), which may include internal, medically supervised, or at-home devices (such as ventilators, neurostim devices), a smart contact, a smart ring, a capillary refill test (e.g., for risk of sores or bruises, diabetic injuries), exercise equipment (e.g., elliptical, mirror, treadmill, bike like peloton, stair stepper, etc.), a phone app that tracks data, an intraoperative data collection device (e.g., vision and robotic information), a chest band (e.g., for respiration and heart rate), or the like. More or fewer individual sensors can be included in the plurality of sensors 16 or another example sensor array. The plurality of sensors 16 can include active sensors and inactive (passive) sensors as further discussed herein.

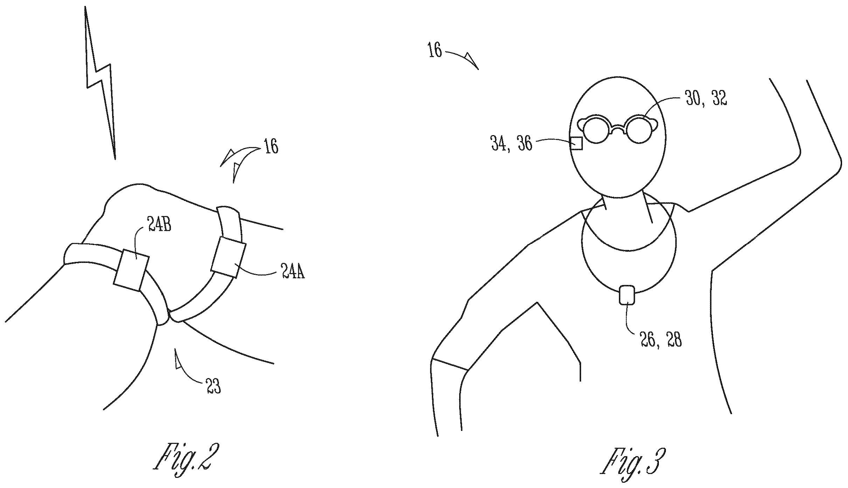

FIG. 2 shows further examples of the plurality of sensors 16 being part of wearable devices. Thus, the wearable device 23 of FIG. 2 includes wearable knee sensors 24A and 24B, which can be used rather than an implanted knee sensor as previously shown. FIG. 3 shows further example of wearable devices that can include the plurality of sensors 16. The wearable devices include a wearable jewelry 26 (e.g. a pendant) having a sensor 28, smart glasses 30 having a sensor 32 and/or an ear worn device 34 having a sensor 36. These devices can be configured using sensors 28, 32 and 36 to sense and gather data regarding user orientation (e.g. posture using IMU) or collect other information (e.g., acceleration, temperature, pH, strain, heart rate force, resistance, strain, gyroscopic, moisture or other data as previously discussed).

The plurality of sensors 16 of FIGS. 1-3 can selectively, such as at the direction of controller 12 (FIG. 1), have an inactive state, and may be later be activated (e.g., upon certain criteria or at a signal from the controller 12 (FIG. 1). Activation can occur such as when data collected from one of the plurality of sensors 16 is insufficient for the analytical predictive model(s) discussed herein and further data for the model is needed, This further data can be collected by a further one or more of the plurality of sensors 16. Data can be collected, compiled, stored, as desired such as dictated by the controller 12 or the plurality of sensors 16 themselves. In an example, some of the plurality of sensors can have two or more different sensor types as part thereof. For example, the implanted sensor 16A of FIG. 1 can include a force sensor, accelerometer, and/or gyroscope. These multiple sensor types may initially be inactive. Applications or sensors including one or more of the plurality of sensors 16 may be activated by a remote device (e.g., the controller 12 as part of a cloud device, a mainframe, or a mobile device, such as a phone) using an app or otherwise communicating with the plurality of sensors 16. Applications implemented by the controller 12 may include circuitry (e.g., a processor, software, firmware, hardwired circuitry, etc.) of the plurality of sensors 16 that is capable of performing various functions. Such functions can be initially disabled, and later enabled when needed.

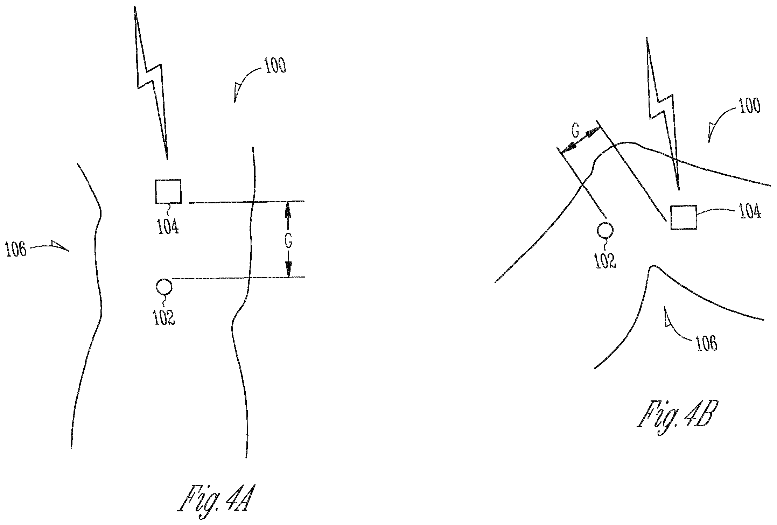

FIGS. 4A and 4B shows an example of an implanted knee sensor 100 having a first component 102 and a second component 104. The first component 102 can be spaced across the knee joint 106 from the second component 104. According to one example, the first component 102 can be a passive device such as a magnet. The second component 104 can be an active device configured to sense position data of the passive device (magnet) and transmit data regarding the passive device (the first component 102). The position data can include a gap G between the first component 102 and the second component (e.g., in extension as shown in FIG. 4A and/or flexion as shown in FIG. 4B). In this manner, changes in the gap G can be identified. Further position data that could be ascertained using the first component 102 and the second component 104 could include range of motion data such as relative movement of the knee (e.g. step count during walking and/or other activity).

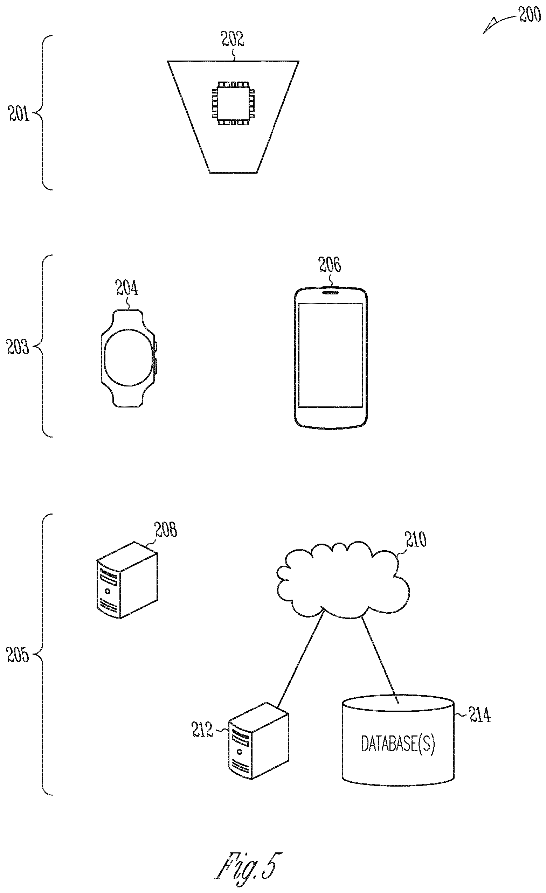

FIG. 5 illustrates a sensor data processing system 200 in accordance with at least one example of this disclosure. The processing system 200 may operate with one or more levels having different processing power, processing capabilities, battery availability, heat requirements, costs, time limits, or the like. A first level 201 may correspond to processing done at a sensor device 202 (e.g., one that is implanted or worn by a user). Sensor device 202 can indeed include a plurality of sensors as contemplated herein. Processing done at the first level 201 may include limited data gathering (e.g., receiving sensor data, storing sensor data, etc.), some compiling, such as adding a timestamp to sensor data, or compiling sensor data into a single file for transmission, or the like. In some examples, processing at the first level 201 may include determining a battery status, memory status, or the like. In an example, processing at the first level 201 may be dependent on timing. For example, in an initial period (e.g., a day, a week, a month, six months), more processing may be done (e.g., more frequent sampling of sensor data, more frequent transmission of sensor data to a remote device, etc.). Then, in a subsequent period, less processing may be done (e.g., less frequent sampling or transmission of sensor data). In an example, sensor data received at the sensor device 202 may be checked for anomalies (e.g., whether the data is within a particular range), such as for temperature, acceleration, etc.

A second level 203 includes devices that may be in proximity to the sensor device 202, such as devices in communication (e.g., direct communication) with the sensor device 202 (e.g., via Bluetooth, Wi-Fi, Wi-Fi direct, via an RFID or other NFC technology, or other FDA approved modality etc.). Devices at the second level 203 may include those with more processing power, capabilities, memory, batteries, or the like than the sensor device 202 of the first level 201, but may still have limitations. Example devices of the second level 203 include a wearable device 204 (which can include sensors) or other internet of things device, a mobile phone 206, a tablet, or the like. Some of these devices may have different limitations than others (e.g., processing power of the mobile device 206 may be greater than that of the wearable device 204).

A third level 205 includes devices with the greatest processing power, capabilities, power, etc. of system 200. Devices in the third level 205 may include a computer 208 (e.g., desktop or laptop), cloud-based devices 210 (e.g., a server 212), which may include access to a database 214, or the like.

Each of the three levels of devices may be used for different types or degrees of processing. For example, the first level 201 may be used for local sensor computing, the second level 203 may be used for middle mobile device computing (e.g., implementing or updating a machine learning model), and the third level 205 may be used for remote server computing (e.g., generating or updating a machine learning model). The tiered architecture of the system 200 may take advantage of remote and local processing capabilities.

In an example, the first level 201 may be used for local processing, such as edge computing on the sensor device. This may include using a basic analytics model uploaded to the sensor device 202. In an example, the sensor device 202 may identify anomalous events independent of mobile device or server interaction. In some examples, the sensor device 202 may enter into an "emergency mode" in real-time (e.g., to shut down due to heat or battery usage, to increase sensor data capture due to identifying a potential issue, or the like).

The second level 203 may be used for to run a more sophisticated analytics model, for example at the mobile device 206. The model may use sensor data from the sensor device 202. In some examples, the model may use additional inputs, such as PROMs, data from the wearable device 204, video recordings of activities, inputs from a user (e.g., a pain rating, a comfort rating, etc.), range of motion information, or the like.

The model may provide interventions in near real time. The model may predict user patterns or user biomechanical changes (and provide intervention for them) over a period of time up to and including years. For example, the model can identity a reduction in user exercise variability (e.g., user went from participating in swimming, running and yoga to only yoga after several years) and can encourage user to participate in other activities in addition to yoga. Factory workers could be encouraged to avoid repetitive activities if possible. The model may also over time identify biomechanical changes that occur in joints and cumulative impact over time such as a reduction in range of motion or user flexibility. The model could also rapidly respond, alert and encourage the user to avoid bad habits such as poor gait, poor posture, or the like. The model could provide exercise regimes, goals or activities to address bad habits, reduction in range of motion, etc. to address these issues before they would have to otherwise be addressed with surgery. The model could also encourage the user to see a medical professional to address conditions identified by the model. For example, the model can determine range of motion has decreased due to a cartilage or ligament tear and notify the user to see a medical professional regarding the possible condition. The model could identify possible underlying patient conditions that can result in excessive or prolonged fatigue, which should be discussed with a medical professional.

The third level 205 may provide server-based processing of a larger population data sets. For example, the mobile device 206 may send data to the cloud 210 (e.g., compiled data that is stripped of personally identifiable information), which may be used with data from other users. The server 212 may develop a model to be pushed out to the mobile device 206 or the sensor device 202. Different models may be developed (e.g., for different user populations, for different devices such as the mobile device 206 and the sensor device 202, for different user timelines such as during a first six months and after six months, or the like). For example, in a first six months or twelve months from when the network was first utilized, more rigorous data collection may occur (e.g., every hour), then after the initial period, the sensor device 202 may transition into a hibernation or check-in mode, which may include obtaining data on demand (e.g., from a signal from the mobile device 206 or the wearable device 204), once a day, once a week, or the like.

The various levels may be used at different time intervals. For example, the ultra-localized processing in the sensor device 202 may be performed multiple times a day, user-specific monitoring may be performed at the mobile device 206 (e.g., daily), and population analytics may be performed at the server 212, such as weekly or monthly. In an example, ultra-localized processing includes cleaning data, generating electrical signals from raw data, processing the data to send summary data to the mobile device 206, optionally some refined outputs, for example not just raw data but compiled data, or the like. The data may be collected at the sensor device 202 for example three times a day, every 5-6 hours, after a first movement read, etc. The data may be sent at each interval to the mobile device 206 or may be held until a sufficient amount of data is stored and sent together.

In an example, anomalies may be alerted using a model at the mobile device 206, the wearable device 204, or the server 212. In response to detecting an anomaly, a change to operation of the sensor device 202 may be sent from the alerting device. The sensor device 202 may change data collection methods, for example changing to a more intense mode (e.g., every fifteen minutes, every hour, etc.), such as for fatigue detection, possible joint, cartilage or ligament damage, etc. In some examples, even when no modeling is done at the second level 203, the devices of the second level 203 may receive data from the sensor device 202 to remove personal data before sending to devices of the third level 205.

In an example, various models may be generated for use with data from the sensor device 202. However, as discussed above, the sensor device 202 has limited resources, such as battery power (which may not be rechargeable in some examples). Data from the wearable device 204 may be readily available and not have battery issues. However, the data from the wearable device 204 may be less accurate than the data from the sensor device 202. In a technique, over time, wearable data may be used as a proxy for implantable sensor data. In this technique, a first model is used for alerting, predicting, or monitoring a user using the implantable sensor data.

A second model or classifier may be used to map wearable data to the implantable sensor data. For example, the wearable data may be labeled with the implantable sensor data or with events (e.g., predictions, alerts, etc.). In one version of the technique, the second model may be used to directly translate wearable data into synthetic implantable sensor data. For example, the wearable data may be input into the second model, and synthetic implantable sensor data may be output from the second model. This synthetic implantable sensor data may then be input into the first model, as if it had been generated by the sensor device 202. In a second version of the technique, the second model may be trained to directly use the wearable data to output alerts, predictions, or the like.

This technique may be used to validate and improve accuracy of data sources, such as the wearable data described above. In some examples, instead of or in addition to the wearable data, mobile device data may be used. The technique may be used to improve accuracy of alerts or predictions for data regarding body posture and/or step counts, for example. In this example, captured gait characteristics of the user (e.g., from the implantable sensor data) may be used to adjust the model of gait stored by measuring devices and used to estimate step count, body posture or other metrics.

Alert detections and predictions may be used to detect or predict abnormal user state or a change in user state requiring intervention based on significant difference from population norm, for example. Such alert detections and predictions can include, for example, warning of fatigue, warning of possible micro-tears to cartilage or ligaments, etc. In some examples, the technique described above may be used to calibrate the wearable device 204 or the mobile device 206. After the second model or classifier is trained or generated, the alerts or predictions may occur without implantable sensor data (e.g., without activating or receiving data from the sensor device 202).

In an example, the wearable data may be used to predict when and what type of implantable sensor data may be useful or needed. For example, the wearable device with sensor(s) may monitor the user to predict how the user's body posture is. However, such data may be insufficient for analytics to be performed and may need to be combined with data collected by the sensor device 202 implanted in the patient.

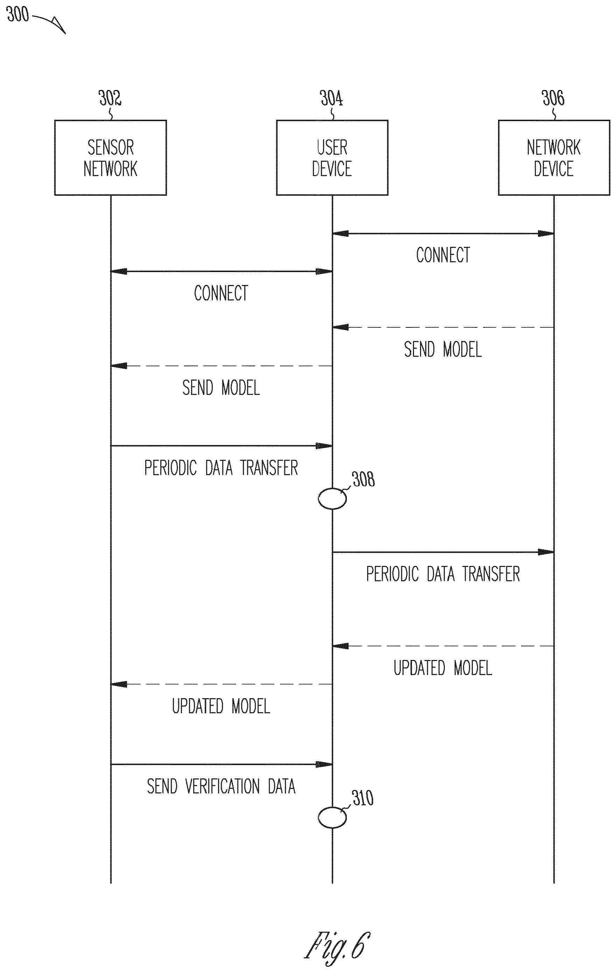

FIG. 6 illustrates a communication diagram 300 for a sensor data processing system in accordance with at least one example of this disclosure. The diagram 300 shows communication between an sensor network 302 (e.g., a plurality of sensors worn and/or implanted in the user), a user device 304 (e.g., a wearable device, a mobile device such as a phone, a computer, etc.), and a network device 306 (e.g., a server, a cloud device, etc.).

The communication diagram 300 illustrates a connection step between the sensor network 302 and the user device 304 and a connection step between the network device 306 and the user device 304. These connection steps may occur in any order, may be initiated by any of the devices, and may occur before or after a surgical procedure to implant the sensor network 302.

In an example, a model may be pre-loaded on the user device 304 or the sensor network 302. In this example, an update to the model may be sent from the network device 306 to the user device 304 (for the user device 304, for the sensor network 302, or for both) or from the user device 304 to the sensor network 302. In another example, a model may be sent from the network device 308 to the user device 304 for loading on the user device 304, the sensor network 302, or both. The user device 304 may send the model to the sensor network 302.

The sensor network 302 may periodically transfer data to the user device 304. After one or more periodic data transfers, the user device 304 may perform analysis on the data at time 308. The analysis may include removing personally identifiable information from the data, running the data through a model to determine whether to output an alert or a prediction, or compiling the data for sending to the network device 306. The user device 304 may periodically transfer data (e.g., forward the same data sent by the sensor network 302, or send data in a different format based on the data sent by the sensor network 302, such as data with personal information removed, compiled data, model results, etc.) to the network device 306. Using information, such as the data sent by the user device 304, data generated by other implantable sensors, wearable sensor data, data collected at the user device 304, or the like, the network device 306 may generate an updated model or an update to a model. The updated model or update to the model may be sent to the user device 304, and then optionally to the sensor network 302 from the user device 304. In some examples, the sensor network 302 may send verification data to the user device 304. The verification data may be used to verify wearable data generated at a wearable device (not shown). The wearable data may be generated as described above with respect to FIG. 5.

A model may use data from the sensor network 302 to provide an alert or prediction related to user activities, user goals or user plan. For example, the model may relate to gait monitoring, posture monitoring, range of motion monitoring, physical activity compliance monitoring, flexibility monitoring, or other goals (which may be set by the patient themselves) or the like.

Predictive analytics may be used to drive a change in sensor configuration dynamically. For example, an output of a model run on the network device 306, the user device 304, or the sensor network 302 may be used to change a parameter of the sensor network 302, such as increasing frequency of data collection, changing type of data collection, or changing how data is compiled or stored. The sensor network 302 may change operation from a normal or standard mode to a more aggressive sensing mode, for example.

An XML-based configuration file may be used to change settings of the sensor network 302. An intelligent data collection system with a tiered architecture for adapting sensor configurations to environmental or user data changes may be used.

In an example, a model may be used for predictive analytics. The model may generate predictive details for a user based on data from the sensor network 302, such as what activity the user is likely to complete, a workout or therapy program, etc. Using the sensor network 302 data, the user may optimize their own individual life plan or health goal.

Predictive analytics may be used to predict user-specific outcomes by considering whether the user is active (e.g., resulting in a higher likelihood the user will need more surgical intervention), or whether the user is likely to over-exercise and damage the body or cause other harm. The sensor network 302 data may be used for refinements, such as to create or modify one or more personalized programs, based on user data, a model, or data or model generated from other users' data (e.g., without personal information). External sensor data (e.g., wearable sensor data) may not be accurate enough to evaluate these types of results and make these types of predictions (e.g., wearable data may only be available to around 5 degrees in range of motion). Some data may be available from the sensor network 302 that includes implanted sensor(s) that is not available in a wearable, such as vibrations, load (e.g., force or pressure), internal temperature, etc.

Predictions may be based on personal goals or targets, and a prediction may be output to a user with a personalized message. For example, a user may set a goal of walking up stairs, playing golf, or participating in yoga daily. The model may generate a prediction based on range of motion, mobility, fatigue, gait and other data from the sensor network 302, etc. This prediction may be used to generate a personalized user message, such as "you're not ready for a full 18 holes of golf but try 9 holes of golf at a manageable pace." The personalized user message may be based on a classification of the user goal into movement, activity or load needs. A plan may be generated to help the user achieve the goal, and data from the sensor network 302 may be used to predict when the goal will be achieved, how the goal may be achieved, and to inform the user what to expect throughout the journey. In an example, real world environmental personal details (e.g., floor plan of house, number of stairs in house, daily walking area, environmental considerations such as hills, city or rural, weather, kids or grandkids, etc.) may be used with the model to generate a prediction. In an example, an initial prediction may be sent to a care team with later predictions sent to a user. In an example, a prediction may be used as a warning, for example if movement indicative of fatigue is detected and the prediction indicates that the user is not ready for strenuous activity, a message may be sent to the user (e.g., via user device 304) warning the user to not attempt strenuous activity.

In an example, the sensor network 302 may be preloaded with a plurality of applications (e.g., configurations, uses, programs, circuitry such as a printed circuit board (PCB), system on a chip (SoC), field-programmable gate array (FPGA), or other integrated circuit or hardware level types of applications, etc.) that are initially not activated. The applications may be validated (e.g., by a regulatory body) before installation in the user. The functions of the applications may be validated before installation, but not activated until after installation in the user. An application may be remotely activated (e.g., using xml or other software instructions, such as in a message from the user device 304). The sensor network 302 may be operably modified to a different programmability capability (e.g., to change the data collection, change the type of data collected, change what sensors collect the data, output, model, etc.), for example changing what the sensor network 302 does or changing data collection type based upon the quality or sufficiency of data collected with one or more sensors that are part of wearable devices.

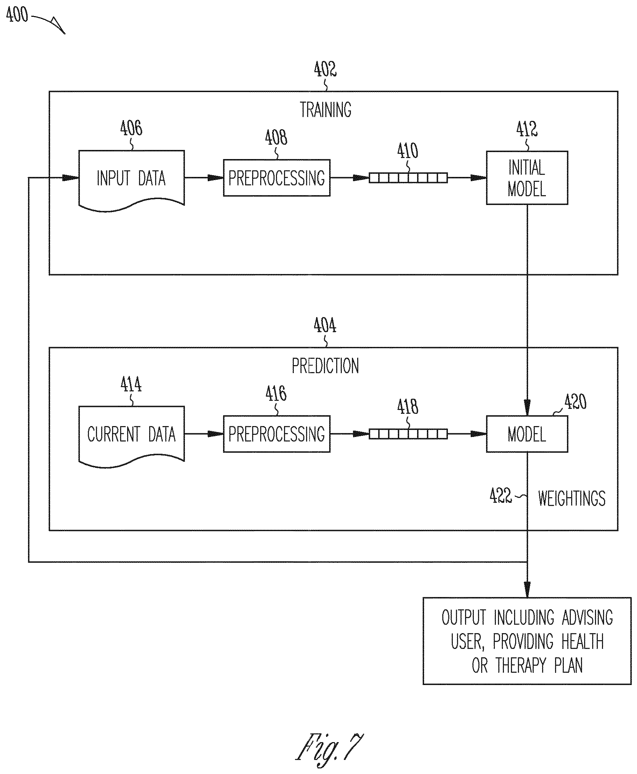

FIG. 7 illustrates a machine learning engine for determining feedback in accordance with some embodiments. The machine learning engine can be employed within a mobile device (e.g., a cell phone) or a computer. A system may calculate one or more weightings for criteria based upon one or more machine learning algorithms. FIG. 7 shows an example machine learning engine 400 according to some examples of the present disclosure.

Machine learning engine 400 utilizes a training engine 402 and a prediction engine 404. Training engine 402 uses input data 406, after undergoing preprocessing 408, to determine one or more features 410. The one or more features 410 may be used to generate an initial model 412, which may be updated iteratively or with future unlabeled data.

The input data 406 may be labeled with an indication, such as user feedback, ambulatory information, range of motion, specific goals (e.g., movement goals, exercise goals, action goals such as lifting items, playing a sport, or driving a car, or the like). In some examples, an outcome may be subjectively assigned to input data, but in other examples, one or more labelling criteria may be utilized that focuses on outcome metrics (e.g., range of motion, pain rating, survey score, a user satisfaction score, such as a forgotten knee score, a WOMAC score, shoulder assessment, hip assessment, or the like).

In the prediction engine 404, current data 414 may be input to preprocessing 416. In some examples, preprocessing 416 and preprocessing 408 are the same. The prediction engine 404 produces feature vector 418 from the preprocessed current data, which is input into the model 420 to generate one or more criteria weightings 422. The criteria weightings 422 may be used to output a prediction, as discussed further below.

The training engine 402 may operate in an offline manner to train the model 420 (e.g., on a server). The prediction engine 404 may be designed to operate in an online manner (e.g., in real-time, at a mobile device, on an implant device, etc.). In other examples, the training engine 402 may operate in an online manner (e.g., at a mobile device). In some examples, the model 420 may be periodically updated via additional training (e.g., via updated input data 406 or based on unlabeled data output in the weightings 422) or user feedback (e.g., an update to a technique or procedure). The initial model 412 may be updated using further input data 406 until a satisfactory model 420 is generated. The model 420 generation may be stopped according to user input (e.g., after sufficient input data is used, such as 1,000, 10,000, 100,000 data points, etc.) or when data converges (e.g., similar inputs produce similar outputs).

The specific machine learning algorithm used for the training engine 402 may be selected from among many different potential supervised or unsupervised machine learning algorithms. Examples of supervised learning algorithms include artificial neural networks, Bayesian networks, instance-based learning, support vector machines, decision trees (e.g., Iterative Dichotomiser 3, C4.5, Classification and Regression Tree (CART), Chi-squared Automatic Interaction Detector (CHAID), and the like), random forests, linear classifiers, quadratic classifiers, k-nearest neighbor, linear regression, logistic regression, and hidden Markov models. Examples of unsupervised learning algorithms include expectation-maximization algorithms, vector quantization, and information bottleneck method. Unsupervised models may not have a training engine 402. In an example embodiment, a regression model is used and the model 420 is a vector of coefficients corresponding to a learned importance for each of the features in the vector of features 410, 418.

Data input sources for the model 420 may include data from the sensors discussed previously including one or more of an implanted sensor device, a watch, a fitness tracker, a wrist-worn device, clothing, a patch worn sensor, a sweat monitor (e.g., electrolytes level), a blood-sugar monitor (e.g., for diabetes), a heart monitor (e.g., EKG or ECG), a heart rate monitor, a pulse oximeter, a stress level monitor (e.g., via an Apple watch), a respiratory rate monitor device, a 'life-alert,' an ear wearable (e.g., for measuring intercranial pressure, such as via the tympanic membrane), a head attached wearable, an ultrasound wearable, a microphone dysphonia device, a smart contact, a smart ring, exercise equipment (e.g., elliptical, mirror, treadmill, bike like peloton, stair stepper, etc.), a phone app that tracks data, an intraoperative data collection device (e.g., vision and robotic information), a chest band (e.g., for respiration and heart rate), video, or the like.

Input data for the model 420 may include user input information, app data, such as from a food app, an exercise tracker, etc., a response to a questionnaire/PROM, video capture (e.g., for range of motion or strength), pain levels, opioid usage, compliance (e.g., with PT or OT or education steps), education data, exercise data, flexibility data, posture data, joint data, demographic or family history information, cognitive tests, BMI, exercise (daily/weekly/monthly), work status (unemployed, working, retired), age, gender, income/wealth status, children, marital status, or the like. Other input information to the model 420 may include clinician side data, a user profile (e.g., demographics, preferences, etc.), a medical history of the user, imaging, an arthritis lab panel, or the like.

Once trained, the model 420 may output a correlated sensor data-based outcome from an input of non-implanted sensor data. In this example, the input data 406 may include sensor data, labeled with wearable data. Each type of data may be saved as a time-series to correlate the sensor data and the wearable data. A separate model may be generated to indicate outcomes for sensor data, for example based on sensor data labeled with outcomes (e.g., user predictions, evaluations, etc.). The model 420 may generate a set of features that correlate the sensor data to the wearable data such that the wearable data may be used to approximate the sensor data. The output of model 420 may include a mapping or feature set to convert wearable data to sensor data such that wearable data may be input to the model 420, which may generate sensor data, which may be used in the separate model to provide an outcome. In this manner, only wearable data may be used to generate outcomes, without the need for or use of sensor data after training. Wearable data can be supplemented as necessary with data from implanted sensor(s).

In another example, the model 420 may predict a user readiness score or indication related to whether a user is ready to perform a particular action, exercise, or specific goal. In an example, this model 420 may be used as the above separate model. This model 420 may be generated using implanted sensor data, wearable data, or any of the other data described above. The output of this model 420 may include advising the user, providing the user with a health or therapy plan or identifying whether or when a user may be able to achieve a specific goal, such as a movement goal, an exercise goal, lifting an item, playing a sport, driving a car, or the like.

In an example, the model 420 may be used to predict whether a user is a high-risk of an adverse event due to joint health, fatigue or the like. When a user is identified as high risk using the model 402, an implanted sensor device may be operated at a higher throughput of obtaining and outputting sensor data.

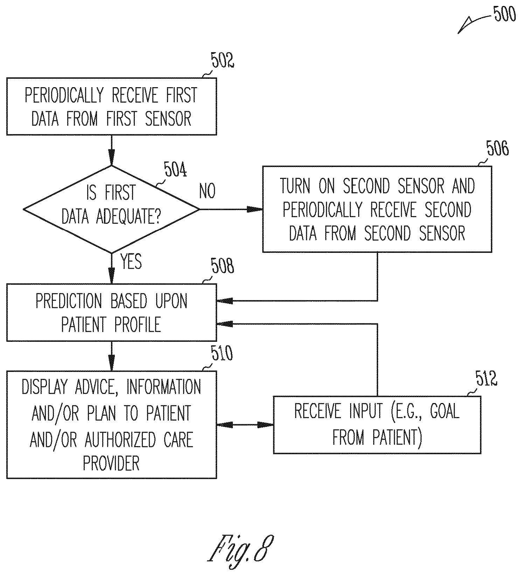

FIG. 8 illustrates a flowchart showing a technique 500 for determining what device to use to process data in a sensor data processing system in accordance with at least one example of this disclosure. The technique 500 may be performed by processing circuitry of a device, such as an implanted sensor device, a wearable device, a mobile device (e.g., a phone, a tablet, etc.), a computer (e.g., a laptop, a desktop, a server, etc.), or the like, locally, on an edge device, or in the cloud.

The technique 500 includes an operation 502 to periodically receive compiled or streamed data generated by a first sensor worn or implanted in the user. The technique 500 includes a determination 504 if first data from the first sensor is adequate to perform analytics on the to provide for a prediction such as a personalized intervention recommendation. If the first data is inadequate to perform analytics on, the technique 500 can turn on 506 a second sensor and can periodically receive compiled or streamed data generated by the second sensor worn or implanted in the user.

The technique 500 includes an operation 508 to perform analytics to output a prediction (e.g., a personalized intervention recommendation). The operation 508 can be run on a mobile device, remote server, etc. In response to the determination at operation 508, various operations may be selected as a next step. For example, the technique 500 can display 510 advice, information, a plan, warning, alert or other data to a user and/or an authorized care provider (e.g., medical professional). The technique 500 can include receiving input from the user (e.g., a patient fitness or life goal, or the like) that can alter the model utilized in the technique 500.

Operation 508 may use user specific information to determine a next step such as the step 510 of display. For example, a posture of the user, a range of motion of the user, a user exercise score, a user gait, user progress towards a goal defined or selected by the user, or the like may be used to determine whether to collect more or less data or more or less types of data from different sensors such as is collected with operations 504 and 506 for modeling in operation 508. In an example, when the user is experiencing fatigue, bad posture, change of range of motion or lowered exercise score, additional information may be obtained by an implanted sensor, for example at operation 506. In this example, the operation 506 may be used to obtain more accurate or diverse amount of information.

The technique 500 at operation 508 can predict an outcome for the user (e.g., the personalized intervention recommendation) by using the first data and/or second data as an input to the local machine learning model. Optionally the operation 508 can receive a predicted outcome from the remote computing device. Operation 508 may occur in response to sending the compiled data to the remote computing device. In an example, before the compiled data is sent to the remote computing device, the compiled data may be sanitized to remove personally identifying information from the compiled data.

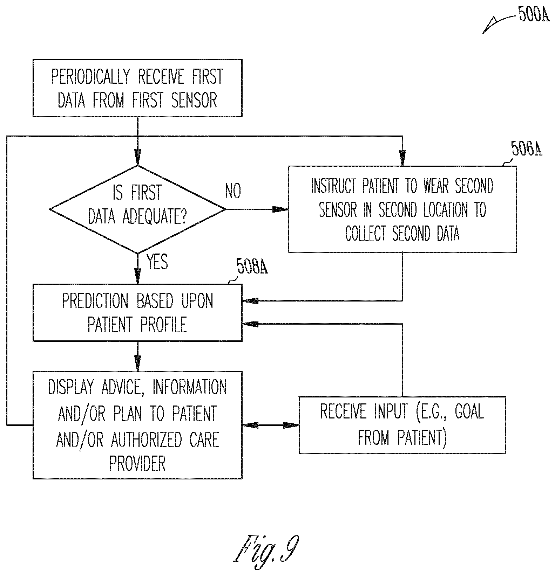

FIG. 9 shows illustrates a flowchart showing an alternative technique 500A for determining what device to use to process data in a sensor data processing system in accordance with at least one example of this disclosure. The technique 500A is similar to that of the technique 500 of FIG. 8 but differs only in that the technique includes an operation 506A to instruct the patient to wear a second sensor in a second location (alternative location to the first sensing location for the first data) to collect second data. This second data can be used in addition to or in alternative to the first data at operation 508A to predict an outcome for the user (e.g., the personalized intervention recommendation) as discussed previously.

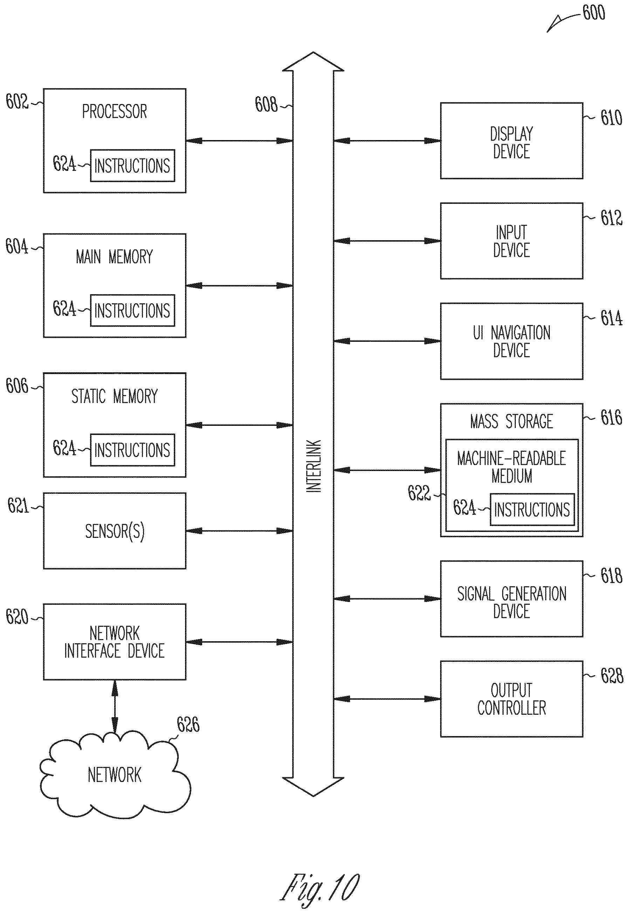

FIG. 10 illustrates a block diagram of an example machine 600 upon which any one or more of the techniques discussed herein may perform in accordance with some embodiments. This example machine can operate some or all of the Orthopedic Intelligence System discussed herein. In some example, the Orthopedic Intelligence System can operate on the example machine 600. In other examples, the example machine 600 is merely one of many such machines utilized to operate the Orthopedic Intelligence System. In alternative embodiments, the machine 600 may operate as a standalone device or may be connected (e.g., networked) to other machines. In a networked deployment, the machine 600 may operate in the capacity of a server machine, a client machine, or both in server-client network environments. In an example, the machine 600 may act as a peer machine in peer-to-peer (P2P) (or other distributed) network environment. The machine 600 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Machine (e.g., computer system) 600 may include a hardware processor 602 (e.g., a central processing unit (CPU), a graphics processing unit (GPU), a hardware processor core, or any combination thereof), a main memory 604 and a static memory 606, some or all of which may communicate with each other via an interlink (e.g., bus) 608. The machine 600 may further include a display unit 610, an alphanumeric input device 612 (e.g., a keyboard), and a user interface (UI) navigation device 614 (e.g., a mouse). In an example, the display unit 610, input device 612 and UI navigation device 614 may be a touch screen display. The machine 600 may additionally include a storage device (e.g., drive unit) 616, a signal generation device 618 (e.g., a speaker), a network interface device 620, and plurality of sensors 621, such as any of those discussed previously (e.g., an IMU, a global positioning system (GPS) sensor, compass, accelerometer, or other sensor). The machine 600 may include an output controller 628, such as a serial (e.g., Universal Serial Bus (USB), parallel, or other wired or wireless (e.g., infrared (IR), near field communication (NFC), etc.) connection to communicate or control one or more peripheral devices (e.g., a printer, card reader, etc.).

The storage device 616 may include a machine readable medium 622 on which is stored one or more sets of data structures or instructions 624 (e.g., software) embodying or utilized by any one or more of the techniques or functions described herein. The instructions 624 may also reside, completely or at least partially, within the main memory 604, within static memory 606, or within the hardware processor 602 during execution thereof by the machine 600. In an example, one or any combination of the hardware processor 602, the main memory 604, the static memory 606, or the storage device 616 may constitute machine readable media.

While the machine readable medium 622 is illustrated as a single medium, the term "machine readable medium" may include a single medium or multiple media (e.g., a centralized or distributed database, and/or associated caches and servers) configured to store the one or more instructions 624. The term "machine readable medium" may include any medium that is capable of storing, encoding, or carrying instructions for execution by the machine 600 and that cause the machine 600 to perform any one or more of the techniques of the present disclosure, or that is capable of storing, encoding or carrying data structures used by or associated with such instructions. Non-limiting machine-readable medium examples may include solid-state memories, and optical and magnetic media.

The instructions 624 may further be transmitted or received over a communications network 626 using a transmission medium via the network interface device 620 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, IEEE 802.16 family of standards known as WiMax^{®}), IEEE 802.15.4 family of standards, peer-to-peer (P2P) networks, among others. In an example, the network interface device 620 may include one or more physical jacks (e.g., Ethernet, coaxial, or phone jacks) or one or more antennas to connect to the communications network 626. In an example, the network interface device 620 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 600, and includes digital or analog communications signals or other intangible medium to facilitate communication of such software.

Each of the following non-limiting examples may stand on its own, or may be combined in various permutations or combinations with one or more of the other examples.

In some aspects, the Examples described herein relate to a system for monitoring health of a user, the system including: a plurality of sensors worn by or implanted in a user, the plurality of sensors includes at least one sensor implanted in the user; a communication unit electronically coupled with the plurality of sensors and receiving data therefrom; a controller electronically coupled with the communication unit, the controller configured to perform predictive analytics on data from the plurality of sensors, wherein the controller is configured to output a personalized intervention recommendation to at least one of the user or a health care provider for use by the user to preemptively address one or more health conditions prior to or in lieu of a surgical intervention for the user.

In some aspects, the Examples described herein relate to a system, wherein the controller is configured to manage collection and transmission of the data by the plurality of sensors including by prioritizing or commanding which one or more of the plurality of sensors collect the data based upon if the data includes sufficient data to perform the predictive analytics thereon.

In some aspects, the Examples described herein relate to a system, wherein the at least one sensor has a small form factor and is implanted with a minimally invasive procedure.

In some aspects, the Examples described herein relate to a system, wherein one or more of the plurality of sensors includes an inertial measurement unit.

In some aspects, the Examples described herein relate to a system, wherein the at least one sensor has an active sensing component implanted at one of a location proximal of a knee joint of the user or distal of the knee joint of the user, wherein the at least one sensor includes a passive component that is implanted in the other of proximal the knee joint or distal of the knee joint, and wherein the at least one sensor measures a gap between the active sensing component and the passive component.

In some aspects, the Examples described herein relate to a system, wherein one or more of the plurality of sensors are mounted on a wearable device that applies a therapy to the user.

In some aspects, the Examples described herein relate to a system, wherein the communication unit electronically shares the personalized intervention recommendation with the health care provider based upon input from the user.

In some aspects, the Examples described herein relate to a system, wherein the controller is located on a mobile device and the personal intervention recommendation is displayed on the mobile device.

In some aspects, the Examples described herein relate to a system, wherein the communication unit at a behest of the user electronically and anonymously shares the personalized intervention recommendation with a network of devices and the controller via communication with the network of devices pairs the user with a second user having a similar personalized intervention recommendation.

In some aspects, the Examples described herein relate to a system, wherein the controller is configured with a machine learning model, the machine learning model is trained using training data collected from a plurality of patients other than the user.

In some aspects, the Examples described herein relate to a method for monitoring health of a user, the method including: receiving, with a networked electronic controller, first data from a first sensor worn by or implanted in the user; determining with the networked electronic controller, if the first data from the sensor provides is sufficient to perform predicative analytics; receiving, with the networked electronic controller, second data from a second sensor worn by or implanted in the user if the first data is insufficient; performing the predictive analytics using the first data or a combination of the first data and the second data; determining a personalized intervention recommendation for the user based upon the predictive analytics; and providing the personalized intervention recommendation to one of the user or a health care provider for use by the user to preemptively address one or more health conditions prior to or in lieu of a surgical intervention for the user.

In some aspects, the Examples described herein relate to a method, further including instructing the user to wear the second sensor if the first data is insufficient to perform predictive analytics.

In some aspects, the Examples described herein relate to a method, further including implanting the first sensor during a minimally invasive procedure.

In some aspects, the Examples described herein relate to a method, further including measuring a gap at a knee joint of the user with the first sensor, wherein the first sensor includes an active sensing component and a passive component, wherein the active sensing component is implanted in one of a location proximal of the knee joint or distal of the knee joint, and wherein the passive component is implanted in the other of the location proximal of the knee joint or distal of the knee joint.

In some aspects, the Examples described herein relate to a method, further including applying a therapy to the user with a device worn by the user.

In some aspects, the Examples described herein relate to a method, further including electronically sharing the personalized intervention recommendation with the health care provider with approval from the user.

In some aspects, the Examples described herein relate to a method, further including: at a behest of the user electronically and anonymously sharing the personalized intervention recommendation with a network of devices; and pairing the user with a second user having a similar personalized intervention recommendation based upon communication with the network of devices.

In some aspects, the Examples described herein relate to a method, wherein the determining personalized intervention recommendation for the user using the predictive analytics includes selecting a machine learning model that is based upon one or more of an overall health of the user including any morbidity conditions, a fatigue level of the user, a pain level identified by the user, a range of motion of the user, a user exercise score, a user progress towards a goal defined by the user, a body orientation of the user based upon at least the first sensor, or a measured physiological characteristic of the user.

In some aspects, the Examples described herein relate to a system for monitoring health of a user, the system including: a first sensor implanted in the user and generating first data; a second sensor worn by the user and generating second data; a communication unit electronically coupled with the first sensor and the second sensor receiving the first data and the second data; and a controller electronically coupled with the communication unit, the controller configured to: perform predictive analytics using at least one of the first data or the second data; determine if one of the first data or the second data includes sufficient data to perform the predictive analytics thereon or if one of the first data or the second data is insufficient to perform the predictive analytics thereon, if one of the first data or the second data is insufficient, combine the first data with the second data to perform the predictive analytics; and determine, based on the predictive analytics, a personalized intervention recommendation for the user for use by the user to preemptively address one or more health conditions prior to or in lieu of a surgical intervention for the user.

In some aspects, the Examples described herein relate to a system, wherein the controller is configured to manage collection and transmission of the first data and the second data by prioritizing or commanding the first sensor or the second sensor to collect the first data or the second data based upon if one of the first data or the second data includes sufficient data to perform the predictive analytics.

In some aspects, the Examples described herein relate to a system, wherein the first sensor has an active sensing component implanted at one of a location proximal of a knee joint of the user or distal of the knee joint of the user, wherein the first sensor includes a passive component that is implanted the other of proximal the knee joint or distal of the knee joint, and wherein the first sensor measures a gap between the active sensing component and the passive component.

In some aspects, the Examples described herein relate to a system, wherein the communication unit electronically shares the personalized intervention recommendation with a health care provider based upon approval from the user.

In some aspects, the Examples described herein relate to a system, wherein the controller is located on a mobile device and the personal intervention recommendation is displayed on and carried by the mobile device.

In some aspects, the Examples described herein relate to a system, wherein the communication unit at the behest of the user electronically and anonymously shares the personalized intervention recommendation with a network of devices and the controller via communication with the network of devices pairs the user with a second user having a similar personalized intervention recommendation.

In some aspects, the Examples described herein relate to a system, wherein the predictive analytics includes selecting a machine learning model that is based upon one or more of an overall health of the user including any morbidity conditions, a fatigue level of the user, a pain level identified by the user, a range of motion of the user, a user exercise score, a user progress towards a goal defined by the user, a body orientation of the user based upon at least the first sensor, or a measured physiological characteristic of the user.

In some aspects, the Examples described herein relate to a system, wherein the personalized intervention recommendation for the user provides an alert to the user advising the user of progress towards a goal.

In some aspects, the Examples described herein relate to at least one machine-readable medium including instructions, which when executed by processing circuitry, cause the processing circuitry to perform operations to: receive a user-specific health goal of a user; receive sensor data generated by a first sensor implanted in or worn by the user; determine, using a predictive analytics model based upon machine learning and the sensor data, a personalized intervention recommendation for the user for the user to preemptively address one or more health conditions prior to or in lieu of a surgical intervention for the user; convert the user-specific health goal to a health plan or therapy plan for use by the user, the health plan or therapy plan based upon the personalized intervention recommendation.

In some aspects, the Examples described herein relate to a at least one machine-readable medium, wherein the sensor data includes first data from the first sensor implanted in the user and second data from a second sensor worn by the user, wherein the instructions which when executed by processing circuitry, cause the processing circuitry to perform operations to: perform predictive analytics using the predictive analytics model and at least one of the first data or the second data; determine if one of the first data or the second data includes sufficient data to perform the predictive analytics thereon or if one of the first data or the second data is insufficient to perform the predictive analytics thereon, if one of the first data or the second data is insufficient, combine the first data with the second data to perform the predictive analytics; and determine, based on the predictive analytics, the personalized intervention recommendation for the user.

In some aspects, the Examples include an apparatus comprising means to implement of any of Examples.

In some aspects, the Examples include a system to implement of any of Examples.

In some aspects, the Examples include a method to implement of any of Examples.

Method and system examples described herein may be machine or computer-implemented at least in part. Some examples may include a computer-readable medium or machine-readable medium encoded with instructions operable to configure an electronic device to perform methods as described in the above examples. An implementation of such methods may include code, such as microcode, assembly language code, a higher-level language code, or the like. Such code may include computer readable instructions for performing various methods. The code may form portions of computer program products. Further, in an example, the code may be tangibly stored on one or more volatile, non-transitory, or non-volatile tangible computer-readable media, such as during execution or at other times. Examples of these tangible computer-readable media may include, but are not limited to, hard disks, removable magnetic disks, removable optical disks (e.g., compact disks and digital video disks), magnetic cassettes, memory cards or sticks, random access memories (RAMs), read only memories (ROMs), and the like.

## Claims

1. A system for monitoring health of a user, the system comprising:
a plurality of sensors (16) including a first sensor (16A)implanted in a user and a second sensor (16B) to be worn by the user;
a communication unit (14) electronically coupled with the plurality of sensors (16) and receiving data therefrom; and
a controller (12) electronically coupled with the communication unit (14), the controller (12) configured to:
determine if first data from the first sensor (16A) is sufficient to perform predicative analytics,
instruct the user to wear the second sensor (16B) if the first data is insufficient to perform predictive analytics,
perform predictive analytics using a combination of the first data from the first sensor (16A) and second data from the second sensor (16B),
determine a personalized intervention recommendation for the user based upon the predictive analytics, and
output the personalized intervention recommendation to at least one of the user or a health care provider for use by the user to preemptively address one or more health conditions prior to or in lieu of a surgical intervention for the user.

2. The system of claim 1, wherein the first sensor (16A) has a small form factor and is configured to be implanted with a minimally invasive procedure and/or one or more of the plurality of sensors (16) comprises an inertial measurement unit.

3. The system of any one of claims 1-2, wherein the first sensor (16A) has an active sensing component implanted at one of a location proximal of a knee joint of the user or distal of the knee joint of the user, wherein the first sensor (16A) includes a passive component that is implanted in the other of proximal the knee joint or distal of the knee joint, and wherein the first sensor (16A) measures a gap between the active sensing component and the passive component.

4. The system of any one of claims 1-3, wherein one or more of the plurality of sensors (16) are mounted on a wearable device that applies a therapy to the user.

5. The system of any one of claims 1-4, wherein the communication unit (14) electronically shares the personalized intervention recommendation with the health care provider based upon input from the user.

6. The system of any one of claims 1-5, wherein the communication unit (14) at a behest of the user electronically and anonymously shares the personalized intervention recommendation with a network of devices and the controller (12) via communication with the network of devices pairs the user with a second user having a similar personalized intervention recommendation.

7. The system of any one of claims 1-6, wherein the controller (12) is configured with a machine learning model, the machine learning model is trained using training data collected from a plurality of patients other than the user, and wherein the machine learning model that is based upon one or more of an overall health of the user including any morbidity conditions, a fatigue level of the user, a pain level identified by the user, a range of motion of the user, a user exercise score, a user progress towards a goal defined by the user, a body orientation of the user based upon at least the first sensor (16A), or a measured physiological characteristic of the user .

8. The system of any one of claims 1-7, wherein the personalized intervention recommendation for the user provides an alert to the user advising the user of progress towards a goal.

9. A method for monitoring health of a user, the method comprising:
receiving, with a networked electronic controller (12), first data from a first sensor (16A) implanted in the user;
determining with the networked electronic controller (12), if the first data from the first sensor (16A) is sufficient to perform predicative analytics;
instructing the user to wear a second sensor (16B) if the first data is insufficient to perform predictive analytics;
receiving, with the networked electronic controller (12), second data from the second sensor (16B);
performing the predictive analytics using a combination of the first data and the second data;
determining a personalized intervention recommendation for the user based upon the predictive analytics; and
providing the personalized intervention recommendation to one of the user or a health care provider for use by the user to preemptively address one or more health conditions prior to or in lieu of a surgical intervention for the user.

10. The method of claim 9, further comprising measuring a gap at a knee joint of the user with the first sensor (16A), wherein the first sensor (16A) includes an active sensing component and a passive component, wherein the active sensing component is implanted in one of a location proximal of the knee joint or distal of the knee joint, and wherein the passive component is implanted in the other of the location proximal of the knee joint or distal of the knee joint.

11. The method of any one of claims 9-10, further comprising electronically sharing the personalized intervention recommendation with the health care provider with approval from the user, and further comprising:
at a behest of the user electronically and anonymously sharing the personalized intervention recommendation with a network of devices; and
pairing the user with a second user having a similar personalized intervention recommendation based upon communication with the network of devices.

## Patentansprüche

1. System zum Überwachen der Gesundheit eines Benutzers, wobei das System Folgendes umfasst:
eine Vielzahl von Sensoren (16) mit einem ersten Sensor (16A), der in einem Benutzer implantiert ist, und einem zweiten Sensor (16B), der von dem Benutzer zu tragen ist;
eine Kommunikationseinheit (14), die elektronisch mit der Vielzahl von Sensoren (16) gekoppelt ist und Daten davon empfängt; und
ein Steuergerät (12), das elektronisch an die Kommunikationseinheit (14) gekoppelt ist, wobei das Steuergerät (12) zu Folgendem konfiguriert ist:
Bestimmen, ob erste Daten von dem ersten Sensor (16A) ausreichen, um prädiktive Analytik durchzuführen,
Anweisen des Benutzers, den zweiten Sensor (16B) zu tragen, wenn die ersten Daten nicht ausreichen, um prädiktive Analytik durchzuführen,
Durchführen von prädiktiver Analytik unter Verwendung einer Kombination der ersten Daten von dem ersten Sensor (16A) und zweiten Daten von dem zweiten Sensor (16B),
Bestimmen einer personalisierten Eingriffsempfehlung für den Benutzer basierend auf der prädiktiven Analytik und
Ausgeben der personalisierten Eingriffsempfehlung an mindestens einen von dem Benutzer oder einem Gesundheitsdienstleister zur Verwendung durch den Benutzer, um präventiv einen oder mehrere Gesundheitszustände vor oder anstelle eines chirurgischen Eingriffs für den Benutzer anzugehen.

2. System nach Anspruch 1, wobei der erste Sensor (16A) einen kleinen Formfaktor aufweist und dazu konfiguriert ist, mit einem minimalinvasiven Verfahren implantiert zu werden und/oder einer oder mehrere der Vielzahl von Sensoren (16) eine Trägheitsmesseinheit umfasst.

3. System nach einem der Ansprüche 1 bis 2, wobei der erste Sensor (16A) eine aktive Erfassungskomponente aufweist, die an einer von einer Stelle proximal eines Kniegelenks des Benutzers oder distal des Kniegelenks des Benutzers implantiert ist, wobei der erste Sensor (16A) eine passive Komponente beinhaltet, die in die andere von proximal des Kniegelenks oder distal des Kniegelenks implantiert ist, und wobei der erste Sensor (16A) einen Spalt zwischen der aktiven Erfassungskomponente und der passiven Komponente misst.

4. System nach einem der Ansprüche 1 bis 3, wobei einer oder mehrere von der Vielzahl von Sensoren (16) an einer tragbaren Vorrichtung montiert sind, die eine Therapie auf den Benutzer anwendet.

5. System nach einem der Ansprüche 1 bis 4, wobei die Kommunikationseinheit (14) die personalisierte Eingriffsempfehlung basierend auf einer Eingabe von dem Benutzer elektronisch mit dem Gesundheitsdienstleister teilt.

6. System nach einem der Ansprüche 1 bis 5, wobei die Kommunikationseinheit (14) auf Geheiß des Benutzers die personalisierte Eingriffsempfehlung elektronisch und anonym mit einem Netzwerk von Vorrichtungen teilt und das Steuergerät (12) über Kommunikation mit dem Netzwerk von Vorrichtungen den Benutzer mit einem zweiten Benutzer mit einer ähnlichen personalisierten Eingriffsempfehlung koppelt.

7. System nach einem der Ansprüche 1 bis 6, wobei das Steuergerät (12) mit einem Modell für maschinelles Lernen konfiguriert ist, wobei das Modell für maschinelles Lernen unter Verwendung von Trainingsdaten trainiert ist, die von einer Vielzahl von anderen Patienten als dem Benutzer gesammelt wurden, und wobei das Modell für maschinelles Lernen, das auf einem oder mehreren von einer allgemeinen Gesundheit des Benutzers, einschließlich etwaiger Morbiditätszustände, einem Ermüdungsniveau des Benutzers, einem von dem Benutzer identifizierten Schmerzniveau, einem Bewegungsbereich des Benutzers, einer Benutzerübungsbewertung, einem Benutzerfortschritt in Richtung eines von dem Benutzer definierten Ziels, einer Körperorientierung des Benutzers basierend auf mindestens dem ersten Sensor (16A) oder einer gemessenen physiologischen Eigenschaft des Benutzers basiert.

8. System nach einem der Ansprüche 1 bis 7, wobei die personalisierte Eingriffsempfehlung für den Benutzer eine Warnung an den Benutzer bereitstellt, die den Benutzer über den Fortschritt in Richtung eines Ziels informiert.

9. Verfahren zum Überwachen der Gesundheit eines Benutzers, wobei das Verfahren Folgendes umfasst:
Empfangen, mit einem vernetzten elektronischen Steuergerät (12), erster Daten von einem ersten Sensor (16A), der in dem Benutzer implantiert ist;
Bestimmen mit dem vernetzten elektronischen Steuergerät (12), ob die ersten Daten von dem ersten Sensor (16A) ausreichen, um prädiktive Analytik durchzuführen;
Anweisen des Benutzers, einen zweiten Sensor (16B) zu tragen, wenn die ersten Daten nicht ausreichen, um prädiktive Analytik durchzuführen;
Empfangen, mit dem vernetzten elektronischen Steuergerät (12), von zweiten Daten von dem zweiten Sensor (16B);
Durchführen der prädiktiven Analytik unter Verwendung einer Kombination der ersten Daten und der zweiten Daten;
Bestimmen einer personalisierten Eingriffsempfehlung für den Benutzer basierend auf der prädiktiven Analytik und
Bereitstellen der personalisierten Eingriffsempfehlung an einen von dem Benutzer oder einem Gesundheitsdienstleister zur Verwendung durch den Benutzer, um präventiv einen oder mehrere Gesundheitszustände vor oder anstelle eines chirurgischen Eingriffs für den Benutzer anzugehen.

10. Verfahren nach Anspruch 9, ferner umfassend das Messen eines Spalts an einem Kniegelenk des Benutzers mit dem ersten Sensor (16A), wobei der erste Sensor (16A) eine aktive Erfassungskomponente und eine passive Komponente beinhaltet, wobei die aktive Erfassungskomponente in eine von einer Stelle proximal des Kniegelenks oder distal des Kniegelenks implantiert ist und wobei die passive Komponente in die andere von der Stelle proximal des Kniegelenks oder distal des Kniegelenks implantiert ist.

11. Verfahren nach einem der Ansprüche 9 bis 10, ferner umfassend das elektronische Teilen der personalisierten Eingriffsempfehlung mit dem Gesundheitsdienstleister mit Zustimmung des Benutzers und ferner umfassend:
auf Geheiß des Benutzers, elektronisches und anonymes Teilen der personalisierten Eingriffsempfehlung mit einem Netzwerk von Vorrichtungen; und
Koppeln des Benutzers mit einem zweiten Benutzer mit einer ähnlichen personalisierten Eingriffsempfehlung basierend auf Kommunikation mit dem Netzwerk von Vorrichtungen.

## Revendications

1. Système permettant la surveillance de la santé d'un utilisateur, le système comprenant :
une pluralité de capteurs (16) comprenant un premier capteur (16A) implanté dans un utilisateur et un second capteur (16B) destiné à être porté par l'utilisateur ;
une unité de communication (14) électroniquement couplée à la pluralité de capteurs (16) et recevant des données en provenance de celle-ci ; et
un dispositif de commande (12) électroniquement couplée à l'unité de communication (14), le dispositif de commande (12) étant configuré pour :
déterminer si les premières données provenant du premier capteur (16A) sont suffisantes pour effectuer une analyse prédictive,
demander à l'utilisateur de porter le second capteur (16B) si les premières données sont insuffisantes pour effectuer une analyse prédictive,
effectuer une analyse prédictive à l'aide d'une combinaison des premières données provenant du premier capteur (16A) et des secondes données provenant du second capteur (16B),
déterminer une recommandation d'intervention personnalisée pour l'utilisateur sur la base de l'analyse prédictive, et
émettre la recommandation d'intervention personnalisée à au moins un parmi l'utilisateur ou un fournisseur de soins de santé en vue d'une utilisation par l'utilisateur pour traiter de façon préventive un ou plusieurs états de santé avant ou à la place d'une intervention chirurgicale pour l'utilisateur.

2. Système selon la revendication 1, ledit premier capteur (16A) présentant un petit facteur de forme et étant conçu pour être implanté avec une procédure mini-invasive et/ou un ou plusieurs parmi la pluralité de capteurs (16) comprenant une unité de mesure inertielle.

3. Système selon l'une quelconque des revendications 1 à 2, ledit premier capteur (16A) comportant un composant de détection actif implanté au niveau d'un parmi un emplacement proximal de l'articulation du genou de l'utilisateur ou un emplacement distal de l'articulation du genou de l'utilisateur, ledit premier capteur (16A) comprenant un composant passif qui est implanté dans l'autre parmi l'emplacement proximal de l'articulation du genou ou l'emplacement distal de l'articulation du genou, et ledit premier capteur (16A) mesurant un espace entre le composant de détection actif et le composant passif.

4. Système selon l'une quelconque des revendications 1 à 3, un ou plusieurs capteurs parmi la pluralité de capteurs (16) étant montés sur un dispositif portatif qui applique un traitement à l'utilisateur.

5. Système selon l'une quelconque des revendications 1 à 4, ladite unité de communication (14) partageant électroniquement la recommandation d'intervention personnalisée avec le fournisseur de soins de santé sur la base de l'entrée de l'utilisateur.

6. Système selon l'une quelconque des revendications 1 à 5, ladite unité de communication (14) à la demande de l'utilisateur partageant électroniquement et anonymement la recommandation d'intervention personnalisée avec un réseau de dispositifs et ledit dispositif de commande (12) par l'intermédiaire d'une communication avec le réseau de dispositifs appariant l'utilisateur avec un second utilisateur ayant une recommandation d'intervention personnalisée similaire.

7. Système selon l'une quelconque des revendications 1 à 6, ledit dispositif de commande (12) étant configuré avec un modèle d'apprentissage automatique, le modèle d'apprentissage automatique étant entraîné à l'aide de données d'entraînement collectées auprès d'une pluralité de patients autres que l'utilisateur, et ledit modèle d'apprentissage automatique qui est basé sur un ou plusieurs parmi une santé globale de l'utilisateur comprenant toutes les conditions de morbidité, un niveau de fatigue de l'utilisateur, un niveau de douleur identifié par l'utilisateur, une plage de mouvement de l'utilisateur, un score d'exercice de l'utilisateur, une progression de l'utilisateur vers un objectif défini par l'utilisateur, une orientation du corps de l'utilisateur basée sur au moins le premier capteur (16A), ou une caractéristique physiologique mesurée de l'utilisateur.

8. Système selon l'une quelconque des revendications 1 à 7, ladite recommandation d'intervention personnalisée pour l'utilisateur fournissant une alerte à l'utilisateur informant l'utilisateur de la progression vers un objectif.

9. Méthode permettant la surveillance de la santé d'un utilisateur, le procédé comprenant :
la réception, avec un dispositif de commande électronique en réseau (12), de premières données provenant d'un premier capteur (16A) implanté dans l'utilisateur ;
la détermination avec le dispositif de commande électronique en réseau (12), pour savoir si les premières données provenant du premier capteur (16A) sont suffisantes pour effectuer une analyse prédictive ;
la demande à l'utilisateur de porter un second capteur (16B) si les premières données sont insuffisantes pour effectuer une analyse prédictive ;
la réception, avec le dispositif de commande électronique en réseau (12), de secondes données en provenance du second capteur (16B) ;
la réalisation de l'analyse prédictive à l'aide d'une combinaison des premières données et des secondes données ;
la détermination d'une recommandation d'intervention personnalisée pour l'utilisateur sur la base de l'analyse prédictive ; et
la fourniture de la recommandation d'intervention personnalisée à un parmi l'utilisateur ou un fournisseur de soins de santé en vue d'une utilisation par l'utilisateur pour traiter de façon préventive un ou plusieurs états de santé avant ou à la place d'une intervention chirurgicale pour l'utilisateur.

10. Méthode selon la revendication 9, comprenant en outre la mesure d'un espace au niveau d'une articulation du genou de l'utilisateur avec le premier capteur (16A), ledit premier capteur (16A) comprenant un composant de détection actif et un composant passif, ledit composant de détection actif étant implanté dans un parmi un emplacement proximal de l'articulation du genou ou un emplacement distal de l'articulation du genou, et ledit composant passif étant implanté dans l'autre parmi l'emplacement proximal de l'articulation du genou ou l'emplacement distal de l'articulation du genou.

11. Méthode selon l'une quelconque des revendications 9 à 10, comprenant en outre le partage électronique de la recommandation d'intervention personnalisée avec le fournisseur de soins de santé avec l'approbation de l'utilisateur, et comprenant en outre :
à une demande de l'utilisateur, le partage électronique et anonyme de la recommandation d'intervention personnalisée avec un réseau de dispositifs ; et
l'appariement de l'utilisateur avec un second utilisateur ayant une recommandation d'intervention personnalisée similaire basée sur la communication avec le réseau de dispositifs.
